# EUROPEAN PATENT APPLICATION

(11) **EP 4 381 936 A1**
(43) Date of publication of application: **12.06.2024**
(21) Application number: 22211377.1
(22) Date of filing: 05.12.2022
(51) Int. Cl.: A01D 78/10

(54) **AGRICULTURAL SYSTEM**

(71) Applicant: CNH Industrial Belgium N.V., 8210 Zedelgem (BE)
(72) Inventor: Eby, Philip, Leola, 17540 (US); Digman, Michael J., Denver, 17517 (US); Pedersen, Torben Gerber, 6200 Aabenraa (DK); Desmet, Stefaan, 9031 Drongen (BE)
(74) Representative: CNH Industrial IP Department

(57) **Abstract**

The present invention relates to an agricultural system (100) comprising: an agricultural rake (102) for a raking operation comprising gathering cut crop material into a windrow, the agricultural rake comprising: a crop condition sensor (110) positioned on the agricultural rake to detect a condition of the cut-crop material prior to, during, and/or after, windrow formation; wherein the agricultural system further comprises: a controller (112) configured to: receive crop condition data (114) from the crop condition sensor; determine a process parameter (116) for a subsequent agricultural process to the raking operation, based on the crop condition data; and output the process parameter.

## Description

### Field

The present disclosure relates to an agricultural system and a method for monitoring the condition of a cut-crop.

### Background

Many agricultural processes involve cutting a crop and leaving at least a portion of the cut-crop in the field for a period of time. For example, the hay making process typically involves cutting a grass or other herbaceous crop and leaving it lying in the field to dry out. Depending upon the dryness, the cut-crop can be collected as silage, haylage or hay by baling. Controlling the drying process is an important aspect of the hay making process. Other agricultural processes involving cut-crop laying in a field may be directed to cut-crop including: alfalfa crop; flax crop; by-products of grain harvesting such as chaff, straw, and residue; and by-products of root-crop harvesting such as haulm topping from potato crops or shredded/mulched leaves from beets.

The present disclosure provides a system and method for monitoring a cut-crop laying in a field to improve the associated agricultural process.

### Summary

According to a first aspect of the present disclosure there is provided an agricultural system comprising:
an agricultural rake for a raking operation comprising gathering cut crop material into a windrow, the agricultural rake comprising:
   a crop condition sensor positioned on the agricultural rake to detect a condition of the cut-crop material prior to, during, and/or after, windrow formation; wherein the agricultural system further comprises:
a controller configured to:
   receive crop condition data from the crop condition sensor;
   determine a process parameter for a subsequent agricultural process to the raking operation, based on the crop condition data; and
   output the process parameter.

The agricultural rake may comprise:
a frame for coupling to an agricultural vehicle; and
a raking unit moveably coupled to the frame and including a plurality of tines.

The process parameter may comprise one or more of: a timing parameter, a selection parameter, and an intensity parameter of the subsequent agricultural process.

The controller may be configured to:
receive position data indicating a position of the agricultural rake from a position sensor; and
determine the process parameter as a position-dependent process parameter based on the crop condition data and the position data.

The agricultural system may further comprise a position sensor for detecting and outputting the position data.

The crop condition sensor may comprise a moisture sensor. The subsequent agricultural process may comprise:
a collection process;
a spraying process;
a tedding process;
a chopping process;
a conditioning process; and/or
a further raking process.

The controller may be configured to receive environmental data and determine the process parameter based on the crop condition data and the environmental data.

The controller may be configured to determine a timing parameter of a subsequent baling process or a subsequent chopping process based on:
moisture data of the crop condition data being less than a lower moisture threshold; and/or
obtaining an expected drying time from a look up table using moisture data of the crop condition data.

The controller may be configured to determine a selection parameter for:
a subsequent tedding process based on moisture data exceeding an upper moisture threshold; or
a subsequent spraying process based on moisture data being less than a lower moisture threshold.

An intensity of the subsequent tedding process may be based on a difference between the moisture data and the upper moisture threshold.

An intensity of the subsequent spraying process may be based on a difference between the moisture data and the lower moisture threshold.

The agricultural rake may comprise a swath guard for intercepting cut-crop material raked by tines of the agricultural rake to form the windrow. The moisture sensor may be positioned on the swath guard.

The agricultural rake may comprise a side delivery rake with one or more swath guards positioned to the side of one or more rotary raking units. The moisture sensor may comprise one or more moisture sensor elements positioned on one or more of the one or more swath guards.

The agricultural rake may comprise a central delivery rake with the swath guard positioned between a first rotary raking unit and a second rotary raking unit. The moisture sensor may comprise:
a first moisture sensor element positioned on a first side of the swath guard facing the first rotary raking unit; and
a second moisture sensor element positioned on a second side of the swath guard facing the second rotary raking unit.

The moisture sensor may comprise a flexible electrode on a surface of the swath guard.

The moisture sensor may be sewn or adhered to the swath guard.

The moisture sensor may comprise a plurality of moisture sensor elements each positioned on a corresponding tine of the agricultural rake.

The controller may be configured to output an abort signal if the moisture data is outside a raking moisture range.

The crop condition sensor may comprise one or more of: a quality sensor, a nutritional value sensor and a yield sensor.

The controller may be configured to:
receive a previous process parameter from an agricultural process previous to the raking operation;
correlate the previous process parameter with the crop condition data; and
determine the process parameter for the subsequent agricultural operation based on the correlation.

The subsequent agricultural process may comprise an agricultural process in a subsequent harvesting cycle. The subsequent agricultural process may comprise: a tilling process, a seeding process, a spraying process, a mowing process, a raking process, a tedding process, a chopping process and/or a collection process.

The controller may be configured to output the process parameter to one or more of:
a user device;
a control centre; and
an agricultural vehicle or machine.

According to a second aspect of the present disclosure there is provided a method for monitoring crop condition comprising:
receiving crop condition data from a crop condition sensor positioned on an agricultural rake to detect a condition of cut crop material during a raking operation, prior to, during, and/or after windrow formation;
determining a process parameter for a subsequent agricultural process to the raking operation, based on the crop condition data; and
outputting the process parameter.

The method may comprise:
receiving a previous process parameter from an agricultural process previous to the raking operation;
correlating the previous process parameter with the crop condition data; and
determining the process parameter for the subsequent agricultural operation based on the correlation.

The method may comprise:
receiving further crop condition data from a plurality of agricultural processes in one or more harvesting cycles;
receiving further process parameters from the plurality of agricultural processes;
determining relationships between the further crop condition data and the further process parameters;
determining the process parameter for the subsequent agricultural process based on the condition data and the determined relationships.

The method may comprise:
receiving environmental data; and
determining the process parameter based on the environmental data.

The crop condition data may comprises: moisture data, nutritional value data and/or yield data.

According to a third aspect of the present disclosure, there is provided a computer program product storing executable instructions for performing the any method disclosed herein.

There may be provided a computer program, which when run on a computer, causes the computer to configure any apparatus, including a circuit, controller, converter, or device disclosed herein or perform any method disclosed herein. The computer program may be a software implementation, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

The computer program may be provided on a computer readable medium, which may be a physical computer readable medium such as a disc or a memory device, or may be embodied as a transient signal. Such a transient signal may be a network download, including an internet download. There may be provided one or more non-transitory computer-readable storage media storing computer-executable instructions that, when executed by a computing system, causes the computing system to perform any method disclosed herein.

### Brief Description of the Drawings

One or more embodiments will now be described by way of example only with reference to the accompanying drawings in which:
Figure 1 illustrates an agricultural system according to an embodiment of the present disclosure;
Figure 2 illustrates an agricultural rake for use in an agricultural system according to an embodiment of the present disclosure;
Figure 3 illustrates a method for monitoring crop condition according to an embodiment of the present disclosure; and
Figure 4 illustrates a further method for monitoring crop condition according to an embodiment of the present disclosure.

### Detailed Description

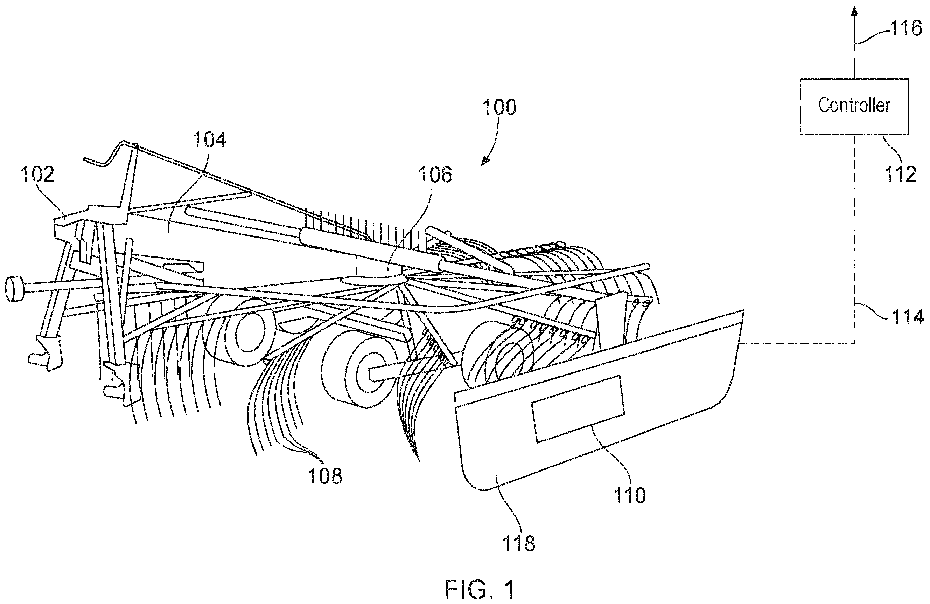

Figure 1 illustrates an agricultural system 100 according to an embodiment of the present disclosure.

The agricultural system 100 includes an agricultural rake 102 for a raking operation. The raking operation comprises raking cut-crop into a windrow. The agricultural rake includes a frame 104 for coupling to an agricultural vehicle (not illustrated). The agricultural vehicle may include a separate vehicle such as a tractor or may include an integral vehicle such that the agricultural rake 102 is self-propelled. The rake 102 also includes a raking unit 106 moveably coupled to the frame 104. The raking unit 106 includes a plurality of tines 108 for engaging with the cut-crop. In this example, the rake 102 is a rotary rake and the raking unit 106 rotates about a vertical axis. The rake 102 also includes a crop condition sensor 110. The crop condition sensor 110 is positioned on the agricultural rake 102 to detect a condition of the cut crop prior to, or during, windrow formation. The system 100 also includes a controller 112. The controller 112 receives crop condition data 114 from the crop condition sensor 110. The controller 112 determines and outputs one or more process parameters 116 for an agricultural process subsequent to the raking operation, based on the crop condition data 114.

By positioning the crop condition sensor 110 on the rake 102 to detect crop condition prior to, or during windrow formation, the system 100 can advantageously obtain a more accurate or granular condition of the cut-crop material than if the crop material was monitored after windrow formation or during a baling process. As a result, the system can provide more precise parameters for the subsequent agricultural process. Providing a condition sensor on a rake advantageously provides condition data between the mowing process and the collection process enabling better management of the collection process or subsequent harvesting cycles, providing a better quality crop.

The one or more process parameters may include any of:
- a timing parameter indicating when the subsequent agricultural process should be performed;
- a selection parameter selecting the specific agricultural process (for example selection of an optional agricultural process, such as a spraying process, conditioning process, additional raking process, windrowing process, baling process, and/or pickup process); and
- an intensity parameter indicating an intensity at which the subsequent agricultural process should be performed.

In some examples, the controller 112 may receive position data indicating a position of the agricultural rake during the raking operation from a position sensor (not shown). In this way, the controller 112 may receive or determine position-dependent condition data comprising an array of condition values with associated position values. The position-dependent condition data can provide a map of the condition of the cut-crop in a field (or fields if the rake is raking more than one field as part of the raking operation). The controller 112 may determine a position-dependent process parameter based on the position data. For example: (i) the controller 112 may determine a position-dependent timing parameter, indicating different times for conducting a subsequent agricultural process on different portions of the field(s); (ii) the controller 112 may determine a position-dependent selection parameter indicating portions of the field(s) in which the subsequent agricultural process should be performed (and portions in which it should not be performed); and/or a position dependent intensity parameter indicating different intensity levels of the subsequent agricultural process for different portions of the field(s).

In some examples, the system 100 may comprise the position sensor, for example the position sensor may be mounted on the frame 104. In some examples, the position sensor may not form part of the system 100, for example the position sensor may form part of a vehicle towing the rake 102. The position sensor may comprise a global positioning system (GPS) sensor and the positioning data may comprise GPS data.

In this example, the crop condition sensor 110 comprises a moisture sensor and the condition data 114 comprises moisture data. In this way, the system 100 can determine a crop readiness for a subsequent agricultural process of the current harvesting operation/cycle, based on the moisture data and output the process parameter accordingly. For example, the subsequent process may include a collection process (e.g. baling or forage harvesting), a chopping process, a spraying process, a tedding process, a conditioning process or a further raking process.

As a first example, the controller 112 may output a timing parameter indicating when a subsequent agricultural process, such as a baling or chopping process, should start. In some examples, the controller 112 may determine that the cut crop material is ready for the subsequent agricultural process if the moisture data is less than an upper moisture threshold and/or greater than a lower moisture threshold. If the controller 112 determines that the cut-crop material is ready for the subsequent process, the controller 112 may output a timing parameter indicating that the subsequent agricultural process can start immediately. In some examples, the controller 112 may use a lookup table to match the moisture data to an expected drying time. The lookup table may provide expected drying time as a function of moisture data. The look-up data may receive one or more environmental parameters (such as the example environmental data described below) and provide the expected drying time as a function of the moisture data and the one or more environmental parameters. The lookup table may also have access to yield data which can affect the drying time, as discussed further below, and determine the expected drying time as a function of the yield data. In some examples, the controller 112 may implement the function of the look up table. The controller 112 may output the timing parameter based on the expected drying time. In examples employing a position sensor, the controller 112 may output a position-dependent timing parameter indicating when to conduct the subsequent agricultural process on different portions of the field(s). For example the timing parameter may indicate that a baling process should occur for a first portion, with moisture data less than the upper dryness parameter, at a first time and a second portion, with moisture data greater than the upper dryness parameter, at a second time, later than the first time. In this way, the cut-crop material in the second portion of the field(s) may dry while the first portion is baled. In other words, the timing parameter may indicate dryer portions of the field(s) to collect first and wetter portions of the field(s) to collect last.

As a second related example, the controller 112 may output a selection parameter indicating that a tedding operation should occur based on the moisture data indicating that the cut-crop material is too wet. The controller 112 may determine that the cut-crop material is too wet if the moisture data is greater than the upper moisture threshold. The controller 112 may also output an intensity parameter indicating an intensity of the tedding based on the moisture data, for example based on a difference between the moisture data and the upper moisture threshold. In examples employing a position sensor, the controller 112 may output a position-dependent selection parameter indicating portions of the cut-crop / field(s) for tedding, and/or a position-dependent intensity parameter indicating a tedding intensity level for different portions of the cut crop material / field(s). The controller may output the position-dependent selection parameter and the position-dependent intensity parameter based on the moisture data as a function of position.

As a third example, the controller 112 may output a selection parameter indicating that a spraying operation should occur based on the moisture data indicating that the cut-crop material is too dry. The controller 112 may determine that the cut-crop material is too dry if the moisture data is lower than a lower moisture threshold. As a further example, the controller 112 may output an intensity parameter indicating an intensity of the spraying (e.g. a flow rate or a concentration of additive etc.) based on the moisture data, for example based on a difference between the moisture data and the lower moisture threshold. In examples employing a position sensor, the controller 112 may output a position-dependent selection parameter indicating portions of the cut-crop / field(s) to be sprayed, and/or a position-dependent intensity parameter indicating a spraying intensity level for different portions of the cut crop material / field(s). For example, the position-dependent intensity parameter may indicate amounts of additive or preservative to be sprayed on different portions of the field (via different flow rates, concentration etc). In this way, value may be provided to a subsequent operation, for example all portions of the field may be baled at the same time with the different amounts of additive/preservative compensating for the moisture variation. The controller may output the position-dependent selection parameter and the position-dependent intensity parameter based on the moisture data as a function of position.

As a fourth example, the controller 112 may output a selection parameter indicating that a baling operation should occur instead of a silage wrapping operation based on the moisture data indicating that the cut-crop material is too dry (with environmental data indicating no forecast precipitation (see below)). Conversely, the controller 112 may output a selection parameter indicating that a silage wrapping operation should occur instead of a baling operation based on the moisture data indicating that the cut-crop material is too wet (with environmental data indicating forecast precipitation (see below)).

In some examples, the controller 112 may receive environmental data such as weather data (e.g., precipitation and humidity data, wind speed, temperature of environment), shadow information (due to trees, slopes etc) and soil moisture data. The weather data may be received as current weather data from a field weather station and/or may be received as current weather and/or future weather forecast from a remote weather data source. The controller 112 may adjust or set the process parameter based on the environmental data. For example, if an expected drying time is four days and the environmental data indicates likely precipitation in three days, the controller 112 may output a timing parameter of a subsequent baling or forage operation of two days and optionally output a selection parameter of a tedding parameter to speed the drying process.

In this example, the agricultural rake 102 includes a swath guard 118 and the moisture sensor 110 is positioned on the swath guard 118. The swath guard 118 is provided to intercept cut crop material raked and projected by the tines 108. The cut-crop material accumulates at the swath guard 118 to form the windrow. By positioning the moisture sensor 110 on the swath guard 118, the moisture sensor 110 can be provided as a contact moisture sensor 110 such as an electrode based moisture sensor, which can advantageously save cost. The moisture sensor 110 can determine a moisture level based on a voltage between two electrodes or based on a voltage between an electrode and ground. By providing the moisture sensor 110 on the swath guard 118 the contact electrode(s) can cover a large surface area such that the moisture of the cut-crop material can be detected with a high resolution and/or accuracy. In some examples, the contact electrode(s) may comprise a contact pattern such as a contact grid that enables the moisture sensor 110 to provide moisture data as a function of location on the swath guard 118. Positioning the moisture sensor 110 on the swath guard 118 can also increase a sensitivity of the moisture sensing because: (i) substantially all of the raked cut-crop matter is intercepted by the swath guard; and (ii) the cut-crop matter tends to slide along the swath guard providing an extended measurement period for the moisture sensor 110.

In some examples, the swath guard 118 may comprise a swath canvas (or swath curtain) comprising a fabric material. In such examples, the moisture sensor may comprise a flexible electrode on the swath canvas. The flexible electrode may be adhered to or woven onto the swath canvas.

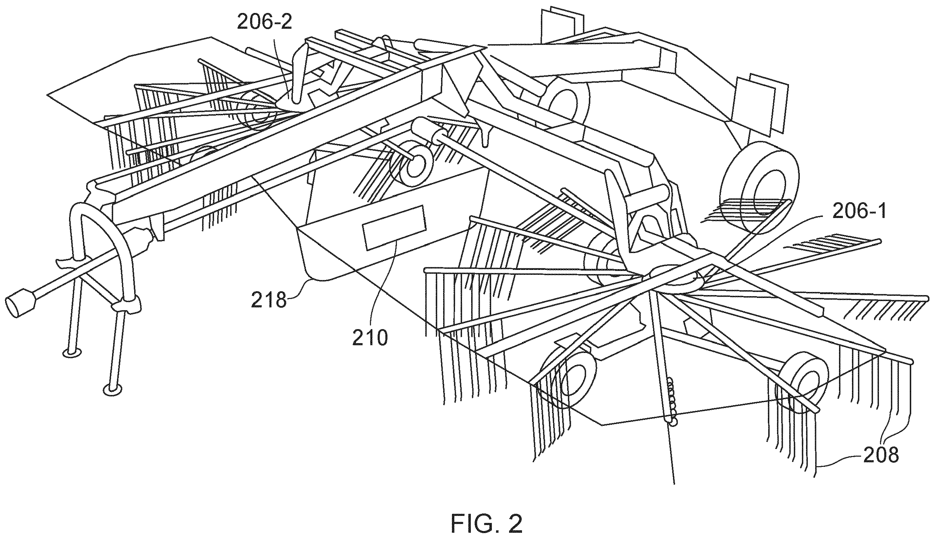

Figure 2 illustrates another agricultural rake 202 for use in an agricultural system according to an embodiment of the present disclosure. Features of Figure 2 which are also present in Figure 1 have been given corresponding reference numbers in the 200 series and will not necessarily be described again here.

In this example, the agricultural rake 202 comprises a double rotary rake with the swath guard 218 positioned between a first rotary raking unit 206-1 and a second rotary raking unit 206-2. The first rotary raking unit 206-1 and the second rotary raking unit 206-2 are substantially similar to the raking unit of Figure 1 and include a plurality of tines 208 configured to rake and project the cut-crop material towards the swath guard 218. In this example the moisture sensor 210 is again positioned on the swath guard 218. As the rake 202 is a double rotary rake with both sides of the swath guard intercepting cut-crop material, the moisture sensor may comprise: a first moisture sensor element positioned on a first side of the swath guard 218 facing the first rotary raking unit 206-1; and a second moisture sensor element positioned on a second side of the swath guard 218 facing the second rotary raking unit 206-2. In this way, the moisture sensor 210 can detect different moisture values from the cut-crop material from each raking unit 206-1, 206-2.

Returning to Figure 1, in other examples (not illustrated) a moisture sensor may be positioned on at least one of the tines 108 or on at least one of the sets of tines. In some examples, a plurality of the tines 108 or a plurality of tine sets may include a moisture sensor. Positioning the moisture sensor on the tines 108 allows the use of a contact moisture sensor. Positioning multiple moisture sensors on multiple tines 108 provides more accurate and higher resolution moisture data.

In yet further examples, the moisture sensor 110 may comprise a non-contact sensor such as a light detecting and ranging (LiDAR) sensor or an infra-red moisture sensor. The contact sensor may be positioned on the swath guard 118 or on the frame 104, in particular at a front of the frame with respect to a direction of forward movement of the rake 102 during the raking operation. Providing a non-contact moisture sensor above the raking operation can provide more accurate and higher resolution moisture data. Providing the moisture sensor at the front of the rake 102 can advantageously provide granular moisture data of cut-crop matter prior to windrow formation.

In some examples, the controller 112 may determine that a moisture level is outside a raking moisture range that is suitable for raking. The controller 112 may output an abort signal indicating that the raking process should be suspended in the current location or aborted altogether. The abort signal may be output to one or more of: a control system of the rake to automatically abort the operation; a user device such as a smart phone of an operator; a control centre monitoring the raking operation; an agricultural vehicle associated with the raking operation, such as a tractor pulling the agricultural rake 102; and an agricultural machine performing (or about to perform) a collection operation (baling, chopping or forage harvesting) which may be arranged to operate after the agricultural rake (for example a few windrows behind).

In addition, or alternatively, to the moisture sensor, the crop condition sensor 110 may comprise one or more other sensor units (which may also be referred to as detectors). The crop condition sensor may include any of: a nutritional value sensor, a yield sensor and a quality sensor. The crop condition sensor may provide the functionality of one or more of the different sensor types using the same detector/sensor unit. For example, all sensors may be provided by a camera based sensor. In other examples, a yield sensor, a nutritional value sensor and/or a quality sensor may be provided by a camera based sensor mounted on a front end of the rake 102 and a moisture sensor may be provided by an electrode based sensor positioned on the swath guard 118. Any of the disclosed functionality of the crop condition sensor 110 may be provided by one or more sensor units.

In some examples, the crop condition sensor 110 may include a nutritional value sensor. The nutritional value sensor may comprise a camera sensor configured to image the cut-crop. The camera sensor may comprise a visible and/or infrared camera or a LiDAR sensor. The controller 112 may receive crop condition data 114 comprising image data from the camera sensor and determine nutritional value data. The controller 112 may use image processing techniques. For example, the controller 112 may determine nutritional value data based on the amount of leaf content on the cut-crop. The controller 112 may determine the amount of leaf content based on leaf content still connected to the stalk of the cut-crop material (rather than leaf content that has been fractured from the stalk of the cut-crop).

In examples with a position sensor, the controller 112 may determine a first portion of the field(s) with a first nutritional value and a second portion of the field(s) with a second nutritional value greater than the first nutritional value. For example, the controller 112 may determine the first nutritional value to be less than a nutritional value threshold and the second nutritional value to be greater than the nutritional value threshold. The higher nutritional value first portion may be suitable for a first feed type (for example a first animal feed type) and the second portion may be suitable for a second feed type. The controller may determine and output a position-dependent timing parameter indicating when a first subsequent collection operation (e.g. baling or foraging) should collect the first portion and when a second subsequent collection process should collect the second portion, based on the position data and the nutritional value data.

The crop condition sensor 110 may include a yield sensor. The yield sensor may comprise a volume sensor for measuring cubic metres of crop per meter (m³/m). The yield sensor may comprise a near infrared (NIR) sensor. The yield sensor may comprise a camera sensor configured to image the cut-crop (and may be provided by a camera sensor providing also the functionality of the nutritional value sensor). The camera sensor may comprise a visible and/or infrared camera or a LiDAR sensor. The controller 112 may receive crop condition data 114 comprising image data from the camera sensor and determine yield data. The controller 112 may use image processing techniques. For example, the controller 112 may determine yield data based on the amount of cut-crop coverage in a field-of-view of the camera. In other examples, the yield sensor may comprise a force sensor or pressure sensor positioned on a part of the rake in contact with the cut-crop, for example the tines 108 or the swath guard 110. For example, the controller 112 may determine yield data of the cut-crop based on a reactive force of the cut-crop on the tines 108 as the raking unit 106 moves the cut-crop at a set rotation speed. The controller 112 may compare the reactive force (optionally together with moisture data which may affect the reactive force) to a look-up table of predetermined yield values.

In examples with a position sensor, the controller 112 may determine a first portion of the field(s) with a first yield value and a second portion of the field(s) with a second yield value greater than the first yield value. The controller may determine and output a position-dependent timing parameter indicating that a subsequent collection operation (e.g. baling or foraging) should collect the first portion before the second portion so that the highest yielding cut-crop material is collected first. This can be particularly advantageous if bad weather, such as heavy precipitation, is forecast.

In example systems comprising both a moisture sensor and a yield sensor, the controller 112 may determine the process parameter for the subsequent agricultural process based on the moisture data and the yield data. For example, the crop yield (the amount of crop which is cut and lays on the field) can have an effect on the drying time of the cut crop: the more crop (and the higher the layer of crop), the longer it will take to dry through. The controller 112 may determine a timing parameter for a subsequent process (e.g. collection or tedding) based on the moisture data and the yield data. The controller may determine a selection parameter for selecting multiple tedding and raking operations to speed up the drying process for high yield crops, based on the moisture data and the yield data.

In some examples (not illustrated), the crop condition sensor 110 may comprise a quality sensor. The quality sensor may be provided by a camera sensor configured to image the cut-crop (and may be provided by a camera sensor providing the functionality of the nutritional value sensor and/or the yield sensor). The controller 112 may receive crop condition data 114 comprising image data from the camera sensor and determine quality data or a quality level of the cut-crop. The quality level / quality data of the cut crop may be based on a level of ash, dirt and/or mould present in the cut-crop material. The controller 112 may use image processing techniques. For example, the controller 112 may implement a trained classifier or other trained machine learning algorithm that has been trained with labelled images of cut-crop material with varying levels of ash, dirt and/or mould. The controller 112 may determine the process parameter based on the quality level of the cut-crop. For example, the controller 112 may determine a selection parameter and a timing parameter for a spraying process (e.g. additive or preservative) to treat the cut-crop if a level of ash, dirt and/or mould exceeds a corresponding threshold. In examples with a position sensor, the controller 112 may determine a position-dependent selection parameter indicating regions of the cut crop material that should not be subject to the subsequent agricultural process (e.g. should not be chopped or baled) or should be subject to the subsequent agricultural process (e.g. should be sprayed). The controller 112 may also output a position-dependent intensity parameter indicating an intensity of spraying for different portions of the field(s).

As described above, the controller 112 can determine one or more process parameters 116 for a subsequent agricultural process in the current harvesting cycle, based on the crop-condition data 114 from the crop condition sensor 110. In some examples, the controller 112 may also determine one or more process parameters of an agricultural process in a subsequent crop harvesting cycle (e.g. a future harvest cycle in a subsequent season or subsequent year).

For example, the controller 112 may determine timing parameters of any process in the subsequent crop harvesting cycle, such as tillage, seeding, spraying (fertiliser, additives, pesticides etc), mowing, raking, tedding etc. based on the moisture data, the quality data, the nutritional data and/or the yield data. In some examples, the timing parameters may comprise a timing with respect to a previous agricultural process, for example "mowing x number of days after spraying." In some examples, the controller 112 may adjust look-up tables of timing parameters for a particular process, for example weather dependent look-up tables that provide different timing parameters depending on a weather forecast. As an example, if the yield data is lower than a yield threshold, the controller 112 may adjust a timing parameter for a mowing operation, such that the crop is not mown too early.

The controller 112 may determine a selection parameter for selecting optional processes in the subsequent crop harvesting cycle, such as spraying, based on the moisture data, the quality data, the nutritional data and/or the yield data. For example, the controller 112 may select a spraying operation based on the quality data being greater than a quality threshold.

The controller 110 may determine an intensity parameter of any process in the subsequent crop harvesting cycle, such as seeding, spraying, mowing and conditioning, raking, tilling, tedding etc based on the moisture data, the quality data, the nutritional data and/or the yield data. For example, the controller 110 may reduce an intensity parameter for a conditioning, tedding or raking process in a subsequent crop harvesting cycle if the nutritional value is less than a nutritional threshold, indicative of leaf fracture due to high intensity processing in the current crop harvesting cycle. As a further example, the controller may alter an intensity of a seeding operation based on the yield value. The intensity of the seeding operation may be varied as a function of position based on the position dependent yield values.

As above, the controller may determine one or more position-dependent process parameters for the process of the subsequent crop harvesting cycle using position data and the condition data. For example, the controller may inhibit or reduce an intensity of a spraying operation in portions of the field(s) with moisture data exceeding an excessive moisture threshold, indicating that these portions are susceptible to excessive moisture, e.g., because of poor drainage.

In some examples, the controller 112 may correlate any of the above described condition data to one or more process parameters of an agricultural process previous to the raking operation. The one or more previous process parameters may be position-dependent process parameters. For example, the controller 112 may correlate a previous fertiliser application process to the nutritional value of the cut-crop. Furthermore, position-dependent fertiliser application may be correlated with position-dependent nutritional value data. As further non-limiting examples: (i) a timing parameter of a previous mowing process may be correlated with a nutritional value of the cut-crop; (ii) an intensity parameter of a previous tedding or a previous raking process may be correlated with nutritional value data; and (iii) a selection parameter of a previous tedding operation may be correlated with moisture data, etc. The controller 112 may receive the process parameters of previous agricultural processes from other agricultural machines.

In some examples, the controller 112 may receive further condition data from: (i) future raking operations of the agricultural rake 102; and/or (ii) other agricultural rakes with crop condition sensors. The controller 112 may also receive: (i) further condition data from one or more further condition sensors associated with one or more agricultural processes previous to the raking operation; (ii) further condition data from one or more further condition sensors associated with one or more agricultural processes subsequent to the raking operation; (iii) one or more process parameters associated with the one or more agricultural processes previous to the raking operation; and (iv) one or more process parameters associated with the one or more agricultural processes subsequent to the raking operation. In this way, the controller 112 can access a rich data set of process parameters and condition data throughout the harvesting cycle, optionally for multiple harvesting cycles (e.g. in the same season if the agricultural machinery is used in multiple fields). The controller may also receive environmental data as described above. The controller 112 may determine correlations between one or more process parameters (and optionally the environmental data) and the condition data to determine the one or more process parameters for the subsequent agricultural process. For example, the controller 112 can monitor the moisture value between successive raking and tedding processes to understand the impact of one or both processes, and/or any associated process parameters, on the drying time of the cut-crop. The controller 112 may then determine and output the process parameters of subsequent raking and/or tedding operations accordingly. As a further example, the controller 112 can monitor the nutritional value data through the harvesting cycle and determine processes that negatively affect the nutritional value. In response, the controller 112 can determine and output one or more parameters of a subsequent agricultural process in response, e.g. an intensity parameter of a subsequent mowing raking, or tedding process.

The controller 112 may implement algorithms such as machine learning algorithms to: determine the relationships between the process parameters, the environmental data and the condition data; and determine and output the process parameters for subsequent agricultural processes based on the condition data, the weather data and the determined relationships. In this way, the controller 112 can monitor correlations between stages of the harvesting cycle and determine optimum process parameters for each stage of the harvesting cycle.

It will be appreciated that the controller 112 may output one or more process parameters for each of one or more agricultural processes subsequent to the raking operation. For example, the controller 112 may output a plurality of timing parameters for a corresponding plurality of subsequent agricultural processes to provide a process schedule. The controller 112 may provide a process schedule for at least a portion of the remaining agricultural processes of the current harvesting cycle or for at least a portion of the agricultural processes of a future harvesting cycle.

In some examples, the controller 112 may be located on the agricultural rake 102. In other examples, the controller 112 may be located at one or more locations remote from the rake 102, for example at a remote monitoring centre. Positioning the controller 112 at a remote monitoring centre may be particularly advantageous for a controller 112 receiving condition data and/or process parameters from multiple agricultural machines. It will be appreciated that the functionality of the controller 112 may be provided by one or more processors. The one or more processors may be located on the agricultural rake 102, at one or more locations remote from the rake 102, or distributed between being located on the rake 102 and located at one or more remote locations.

The controller 112 may output the one or more process parameters to one or more of: a user device, such as a smart phone or tablet of an operator, a remote monitoring centre and an agricultural vehicle or machine. The controller 112 may output the one or more process parameters as an information signal for informing an operator of a suggested processing parameter for the subsequent agricultural process (e.g. a suggested time to perform a baling process, which may be specified in hours, days etc.). The controller 112 may output the one or more process parameters to an agricultural machine as a control setting for automatically setting the process parameter of the agricultural process or as a control signal for automatically controlling the agricultural machine. For example, the controller 112 may output a position-dependent selection signal as a selective enable / inhibit control signal to a baler based on the moisture level of the cut-crop material in the field(s).

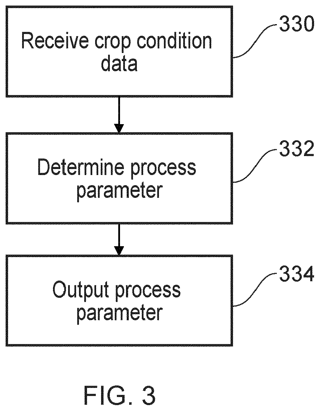

Figure 3 illustrates a method for monitoring crop condition according to an embodiment of the present disclosure.

A first step 330 of the method comprises receiving crop condition data from a crop condition sensor positioned on an agricultural rake to detect a condition of cut crop material during a raking operation, prior to, or during, windrow formation.

A second step 332 comprises determining a process parameter for a subsequent agricultural process to the raking operation, based on the crop condition data.

A third step 334 comprises outputting the process parameter.

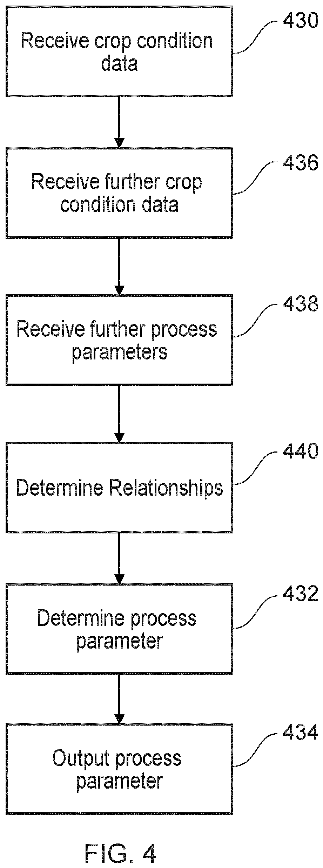

Figure 4 illustrates a further method for monitoring crop condition according to an embodiment of the present disclosure.

Figure 4 comprises the same steps as the method of Figure 3 and the additional steps of:
- Receiving 436 further crop condition data from a plurality of agricultural processes in one or more harvesting cycles. The plurality of agricultural processes may comprise agricultural processes previous to and/or subsequent to the raking operation and/or agricultural processes from a different harvesting cycle.
- Receiving 438 further process parameters from the plurality of agricultural processes.
- Determining 440 relationships between the further crop condition data and the further process parameters. Determining the relationships may be performed using one or more algorithms such as machine learning algorithms.

The step of determining 432 the process parameter for the subsequent agricultural process comprises determining the process parameter based on the condition data and the determined relationships.

As disclosed herein the terms moisture data, quality data, nutritional value data and yield data may comprise one or more respective values. Position dependent moisture data, quality data, nutritional value data and yield data may comprise an array of respective values each with an associated position value.

Throughout the present specification, the descriptors relating to relative orientation and position, such as "horizontal", "vertical", "top", "bottom" and "side", are used in the sense of the orientation of the rake or system as presented in the drawings. However, such descriptors are not intended to be in any way limiting to an intended use of the described or claimed invention.

It will be appreciated that any reference to "close to", "before", "shortly before", "after" "shortly after", "higher than", or "lower than", etc, can refer to the parameter in question being less than or greater than a threshold value, or between two threshold values, depending upon the context.

## Claims

1. An agricultural system (100) comprising:
an agricultural rake (102) for a raking operation comprising gathering cut crop material into a windrow, the agricultural rake comprising:
a crop condition sensor (110) positioned on the agricultural rake (102) to detect a condition of the cut-crop material prior to, during, and/or after, windrow formation; wherein the agricultural system further comprises:
a controller (112) configured to:
receive crop condition data (114) from the crop condition sensor (110);
determine a process parameter (116) for a subsequent agricultural process to the raking operation, based on the crop condition data (114); and
output the process parameter (116).

2. The agricultural system (100) of claim 1, wherein the process parameter (116) comprises one or more of: a timing parameter, a selection parameter, and an intensity parameter of the subsequent agricultural process.

3. The agricultural system (100) of claim 1 or claim 2, wherein the controller (112) is configured to:
receive position data indicating a position of the agricultural rake (102) from a position sensor; and
determine the process parameter (116) as a position-dependent process parameter based on the crop condition data (114) and the position data.

4. The agricultural system (100) of any preceding claim, wherein the crop condition sensor (110) comprises a moisture sensor and the subsequent agricultural process comprises:
a collection process;
a spraying process;
a tedding process;
a chopping process;
a conditioning process; and/or
a further raking process.

5. The agricultural system (100) of claim 4, wherein the agricultural rake (102) comprises a swath guard (118) for intercepting cut-crop material raked by tines (108) of the agricultural rake to form the windrow, wherein the moisture sensor is positioned on the swath guard.

6. The agricultural system (100) of claim 5, wherein the moisture sensor comprises a flexible electrode on a surface of the swath guard (118).

7. The agricultural system (100) of claim 4, wherein the moisture sensor comprises a plurality of moisture sensor elements each positioned on a corresponding tine (108) of the agricultural rake (102).

8. The agricultural system (100) of any of claims 4 to 7, wherein the controller (112) is configured to output an abort signal if the moisture data is outside a raking moisture range.

9. The agricultural system (100) of any preceding claim, wherein the crop condition sensor (110) comprises one or more of: a quality sensor, a nutritional value sensor and a yield sensor.

10. The agricultural system (100) of any preceding claim, wherein the subsequent agricultural process comprises an agricultural process in a subsequent harvesting cycle.

11. The agricultural system of any preceding claim, wherein the controller (112) is configured to output the process parameter (116) to one or more of:
a user device;
a control centre; and
an agricultural vehicle or machine.

12. A method for monitoring crop condition comprising:
receiving crop condition data (114) from a crop condition sensor (110) positioned on an agricultural rake (112) to detect a condition of cut crop material during a raking operation, prior to, during, and/or after windrow formation;
determining a process parameter (116) for a subsequent agricultural process to the raking operation, based on the crop condition data (114); and
outputting the process parameter (116).

13. The method of claim 12, further comprising:
receiving a previous process parameter from an agricultural process previous to the raking operation;
correlating the previous process parameter with the crop condition data (114); and
determining the process parameter (116) for the subsequent agricultural operation based on the correlation.

14. The method of claim 12 or claim 13, further comprising:
receiving further crop condition data from a plurality of agricultural processes in one or more harvesting cycles;
receiving further process parameters from the plurality of agricultural processes;
determining relationships between the further crop condition data and the further process parameters;
determining the process parameter (116) for the subsequent agricultural process based on the condition data (114) and the determined relationships.

15. The method of any of claims 12 to 14 wherein the crop condition data (114) comprises: moisture data, nutritional value data and/or yield data.
